# FASCICULE DE BREVET EUROPEEN

(11) **EP 0 763 043 B1**
(45) Date de publication et mention de la délivrance du brevet: **23.09.1998**
(21) Numéro de dépôt: 95921739.9
(22) Date de dépôt: 10.05.1995
(51) Int. Cl.: C07D 501/22

(54) **PROCEDE POUR LA PREPARATION DU CEFIXIME TRIHYDRATE**
VERFAHREN ZUR HERSTELLUNG VON CEFIXIME TRIHYDRAT
PROCESS FOR THE PREPARATION OF TRIHYDRATED CEFIXIME

(30) Priorité: 03.06.1994 CH 1751/94
(43) Date de publication de la demande: 19.03.1997
(73) Titulaire: MARCHAM TRADING & INVESTMENT LTD., Dublin 4 (IE)
(72) Inventeur: PICORNELL DARDES, Carlos, E-Madrid (ES)
(74) Mandataire: Dragotti, Gianfranco
(86) Numéro de dépôt international: EP9501759
(87) Numéro de publication internationale: WO9533753

(56) Documents cités:
- EP-A- 0 030 630
- CHEMICAL ABSTRACTS, vol. 109, no. 12, 19 Septembre 1988, Columbus, Ohio, US; abstract no. 98626a, page 349 ;colonne R ; & FED. REGST., vol.53, no.124, 28 Juin 1988, ROCKVILLE pages 24256 - 24256 UNITED STATES FOOD AND DRUG ADMINISTRATION
- THE JOURNAL OF ANTIBIOTICS, vol.38, no.12, Décembre 1985, TOKYO pages 1738 - 1751 HIDEAKI YAMANAKA ET AL cité dans la demande

## Description

La présente invention concerne un procédé pour la préparation du céfixime trihydraté ainsi que de nouveaux intermédiaires 3-céphème.

Céfixime est la Denomination Commune Internationale d'un nouvel antibiotique cephalosporinique actif par voie orale de formule (A) 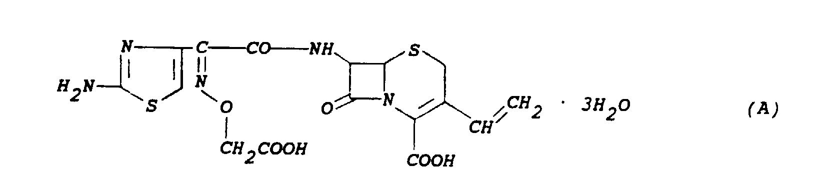 décrit dans The Journal of Antibiotics, 1985, 38, 1738-1751.

La publication susdite et le document EP 30.630 décrivent une série de procédés pour la préparation du céfixime, qui conduisent tous à un intermédiaire dans lequel les deux groupes carboxy présents dans sa formule sont protégés de façon différente.

Plus particulièrement, selon la méthode apparemment la plus utilisée dans la préparation du céfixime, le groupe carboxy en position 4 du cycle céphème est protégé sous forme d'ester benzhydrylique, alors que le groupe carboxylique de l'acide acétique lié au groupe oxyimino est protégé sous forme d'ester tert-butylique. L'élimination simultanée des groupes tert-butylique et benzhydrylique est effectuée, suivant les documents susdits, par hydrolyse avec des acides inorganiques ou organiques, par exemple avec acide chlorhydrique, acide trifluoracétique ou avec des mélanges d'acide chlorhydrique et acide formique.

La publication citée ci-dessus décrit aussi la double élimination par traitement avec de l'acide trifluoracétique et anisole avec un rendement de 34,1%.

Le document EP 30.630 décrit l'élimination des groupes protecteurs par traitement avec une mélange acide chlorhydrique/acide formique mais, bien que les rendements indiqués soient élevés, à la fin de l'hydrolyse on obtien une mélange de produits ayant la configuration syn- et anti- au niveau du groupe oxyimino. Ces produits ne peuvent pas être separés entre eux.

Il est donc très souhaitable de pouvoir disposer d'une méthode permettant d'obtenir avec des rendements élevés un céfixime presqu'exempt de son isomère anti-.

Il a été maintenant trouvé qu'en faisant réagir l'acide (Z)-2-(2-aminothiazol-4-yl)-2-(tert-butoxycarbonylméthoxyimino)acétique N-protégé avec le 7-amino-3-vinyl-3-céphème-4-carboxylate de tert-butyle on obtien un nouvel intermédiaire particulièrement intéressant.

Il a été également trouvé, de façon surprenante, que par traitement de cet intermédiaire, éventuellement N-deprotégé, avec chlorure d'aluminium et anisole, on obtient le céfixime, pratiquement exempt de son isomère anti-, avec des rendements très élevés.

Il a été enfin trouvé qu'à partir de réactifs convenablement choisis, il est possible d'obtenir le céfixime pure avec des rendements globaux supérieurs à 80% et même à 90%.

Ainsi, selon un de ses aspects, la présente invention a pour objet un procédé pour la préparation du céfixime trihydraté de formule (A) caractérisé en ce que
(a) on traite un dérivé fonctionnel de l'acide (Z)-2-(2-aminothiazol-4-yl)-2-(tert-butoxycarbonylméthoxyimino)acétique N-protégé de formule (I) 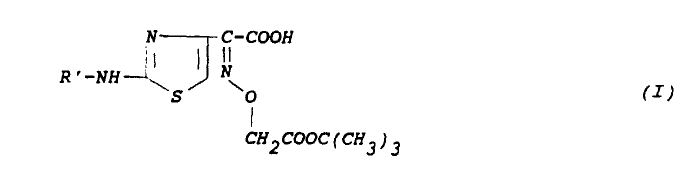 dans laquelle R' représente un groupe N-protecteur, avec le 7-amino-3-vinyl-3-céphème-4-carboxylate de tert-butyle de formule (II) 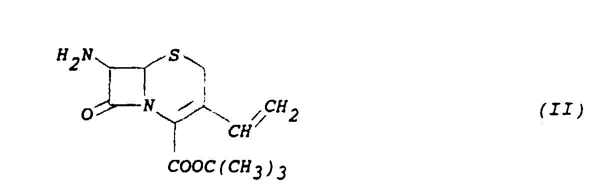 ou avec un de ses sels; et
(b) après l'élimination éventuelle du groupe protecteur du produit ainsi obtenu de formule (III) 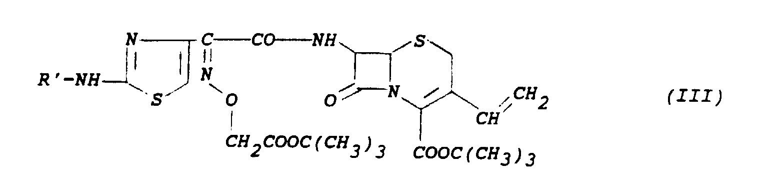 dans laquelle R' est tel que défini ci-dessus, on traite le composé ainsi obtenu de formule (IV) 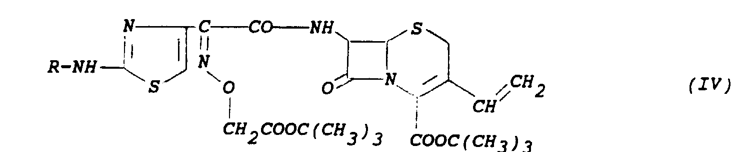 dans laquelle R représente l'hydrogène ou un groupe N-protecteur, avec trichlorure d'aluminium et anisole.

Dans l'étape (a), le dérivé fonctionnel de l'acide (I) peut être l'acide même, convenablement activé, par exemple, avec la dicyclohexylcarbodiimide, le 1-hydroxybenzotriazole ou le mercaptobenzothiazole; le chlorure de l'acide (I); un anhydride mixte, par exemple avec un acide sulfonique ou avec le monoester éthylique de l'acide carbonique. Le chlorure de l'acide (I) peut être aisemement préparé et utilisé "in situ" à partir de l'acide même par une réaction de Vilsmeyer avec oxychlorure de phosphore et N,N-diméthylformamide en quantités à peu près équimoléculaires. Un anhydride mixte particulièrement avantageux peut être préparé à partir de l'acide (I) par réaction avec un acide sulfonique de formule (V)

R"-SO₂Cl (V)

dans laquelle R" représente un groupe alkyle de 1 à 4 atomes de carbone ou un groupe phényle, non substitué ou substitué, de préférence avec un groupe méthyle, et on traite après l'anhydride mixte ainsi obtenu de formule (VI) 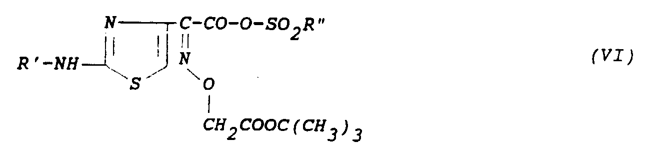 dans laquelle R' et R" sont tels que définis ci-dessus, avec le 7-amino-3-vinyl-3-céphème-4-carboxylate de tert-butyle de formule (II). La réaction de formation de l'anhydride mixte par traitement du composé (II) avec le composé (V) est effectuée dans un solvant organique de type éthéré ou aprotique polaire ou halogéné comme tétrahydrofurane, dioxane, N,N-diméthylformamide, N,N-diméthylacétamide, dichlorométhane ou mélanges de ces solvants, à température ambiante. La réaction est très rapide.

Le traitement de l'anhydride mixte (VI) ainsi obtenu avec le composé (II) peut être effectué "in situ" à une température entre -10°C et 0°C en présence d'une base organique. En général la base organique est introduite goutte à goutte dans le mélange de réaction contenant l'anhydride mixte, à la température susdite et, après une agitation de 1ö3 heures, le composé de formule (II) est ajouté, avantageusement sous forme d'un de ses sels, de préférence avec un acide sulfonique. La réaction a lieu en 1ö3 heures, toujours à la température susdite, de préférence entre -5°C et 0°C.

Lors que le dérivé fonctionnel de l'acide (I) de départ est le chlorure obtenu selon la réaction de Vilsmeyer, l'acide libre est traité avec oxychlorure de phosphore et N,N-diméthylformamide en quantités à peu près equimoléculaires dans un solvant tel que le tétrahydrofurane, le dioxane, le dichlorométhane. Le composé (II) est utilisé de préferénce sous forme de son p-toluène sulfonate.

Le nouvel intermédiaire (III) ainsi obtenu est isolé suivant les méthodes conventionelles par neutralisation avec une base inorganique ou organique, par exemple carbonate sodique ou potassique, triéthylamine et similaires et par évaporation du solvant de la phase organique.

Le composé de formule (I) utilisé comme produit de départ a sur l'amine thiazolique un groupe N-protecteur qui peut être n'importe quel groupe qui sert à protéger le groupe amino dans les réactions dans lesquelles le dit groupe doit être préservé. N'importe quel groupe protecteur qu'on peut utiliser dans la chimie des cephalosporines du type céfixime, contenant un groupe aminothiazolique, peut être representé par R' par exemple, un groupe formyle, trichloroacétyle, trityle, benzyloxycarbonyle ou tert-butoxycarbonyle.

Pour l'utilisation du composé de formule (III) comme intermédiaire, le groupe N-protecteur R' peut être eliminé selon les méthodes connues en littérature. Ainsi, par exemple, le groupe formyle peut être éliminé par hydrolyse acide, comme aussi les groupes trichloroacétyle, trityle, benzyloxycarbonyle et tert-butoxycarbonyle, tandis que les groupes tert-butyliques sont conservés pour être éliminés dans un deuxième temps. On obtient ainsi un composé de formule (IV) dans laquelle R est l'hydrogène.

Dans l'étape (b) on élimine éventuellement du composé de formule (III) le groupe N-protecteur R' pour obtenir le composé de formule (IV), dans laquelle R est hydrogène, qui est par la suite traité avec chlorure d'aluminium et anisole.

L'élimination du groupe N-protecteur R' est effectué comme indiqué ci-dessus. En particulier, le groupe formyle est enlevé par hydrolyse acide dans un solvant alcoolique et le produit N-deprotégé (IV, R=H) est isolé par neutralisation avec une base et précipitation avec de l'eau.

Selon un mode opératoire préférentiel, dans l'étape (b):
(b₁) le composé de formule (III), dans laquelle R' est formyle, est soumis à une N-déprotection par hydrolyse avec un acide dissous dans un alcool, de préférence acide chlorhydrique dans le méthanol, pour obtenir un composé de formule (IV) où R est l'hydrogène; et
(b₂) ce dernier est soumis à l'action du trichlorure d'aluminium et de l'anisole pour obtenir le céfixime trihydraté.

Si le groupe amino du thiazole est protégé avec un groupe benzyloxycarbonyle, la deprotéction peut avoir lieu par traitement avec chlorure d'aluminium et anisole. Dans ce cas, la N-deprotéction et la double élimination des groupes tert-butyliques estérifiant les carboxyles du composé (IV) ont lieu en même temps et on peut isoler directement le céfixime.

La réaction avec chlorure d'aluminium et anisole est effectuée de préférence en utilisant de trois à six moles de AlCl₃ par mole de composé (I) et le même anisole comme solvant. On peut également utiliser l'anisole en quantités molaires à peu près égales à celle du chlorure d'aluminium en utilisant un solvant de préférence halogéné comme chlorure de méthylène ou 1,1,1-trichloroéthane. La réaction est conduite à une température de +5ö+15°C pendant 30ö120 minutes et le céfixime ainsi obtenue est isolée selon les techniques ordinaires. Le rendement en céfixime trihydraté est très élévé, en général supérieur à 90%.

Les composés de départ corrispondant aux formules (I) et (II) sont connus en littérature. Plus particulièrement, les composés de formule (I) peuvent être preparés à partir d'un ester de l'acide 2-aminothiazol-4-ylglyoxylique par protéction du groupe amino avec le radical R', saponification de l'ester et traitement de l'acide 2-aminothiazol-4-ylglyoxylique N-protégé ainsi obtenu avec la 0-tert-butoxycarbonylméthoxyamine. L'acide (Z)-2-(2-formamidothiazol-4-yl)-2-(tert-butoxycarbonylméthoxyimino)acétique (I, R' = formyle) est décrit dans The Journal of Antibiotics 1983, 36, 846-853. Le composé de départ correspondant à la formule (II) est préparé suivant la méthode décrite dans US 4,107,431 et est utilisé pour la réaction avec le composé (IV) sous forme de un de ses sels, par exemple le chlorhydrate, méthanesulfonate ou, de préférence, le p-toluènesulfonate.

Les nouveaux composés de formule (IV) constituent les intermédiairés clé du procédé de la présente invention. Ainsi, selon un autre de ses aspects, la présente invention concerne un composé de formule (IV) ci-dessus, dans laquelle R est tel que défini ci-dessus. Les composés de formule (IV) dans laquelle R est l'hydrogène, un groupe formyle ou un groupe benzyloxycarbonyle sont particulièrement préférés.

Les exemples suivants illustrent l'invention sans, toutefois, la limiter. Dans la PREPARATION et dans les Exemples qui suivent, les réactions sont contrôlées par HPLC dans les conditions suivantes: colonne: Hypersi®100 C-18 (Shandon) (5µ, 250 x 4,6 mm); phase mobile: tampon phosphate 0,05 M (pH = 4,5/acétonitrile à 10% dans l'eau (système gradient) - 2 ml/minute; λ= 254 nm.

Les spectres RMN ont été déterminés à 300 MHz.

La résine Amberlite®XAD 1180 est fabriquée par la société Rohm and Haas Co. (Etats-Unis d'Amérique). Dicalite®408 et Diafioc®40 sont des adjuvants de filtration fournis par la société Dicalite Europa Sud (Italie).

### PREPARATION

### Acide (Z)-2-(benzyloxycarbonylaminothiazol-4-yl)-2(tert-butoxycarbonylméthoxyimino)acétique monohydraté

A une solution de 5,7 g (0,0186 m) d'acide 2-(benzyloxycarbonylaminothiazol-4-yl)glyoxylique in 60 ml de N,N-dimethylacétamite on ajoute, à 20°C, 3,6 g (0,024 m) d'aminooxyacétate de tert-butyle. On agite le mélange 90 minutes à environ 25°C (on peut contrôler par HPLC la totale consommation du produit de départ), puis on verse lentement le mélange réactionnel dans 60 ml d'eau, on agite pendant une heure à 20ö25°C, on refroidit à +5°C et on maintient le mélange à cette température pendant 30 minutes. Le mélange est enfin filtré, le produit est lavé à l'eau et séché sous vide à 40°C. On obtient ainsi 7,2 g (85%) du composé indiqué dans le titre. P.f.: 134ö135°C. IR: νₘₐₓ: 3200, 2980, 2934, 1740, 1715, 1560 cm⁻¹.
¹H-RMN (DMSO-d₆): δ (p.p.m.) 1,45 (9H, s, -CMe₃), 4,66 (2H, s, -OCH₂CO), 5,30 (2H, s, -O-CH₂-C₆H₅), 7,43 (5H, s, protons aromatiques), 7,5 (1H, s, proton thiazole).

### EXEMPLE 1

A un mélange de 12 ml de tétrahydrofurane et 1,15 ml (0,0149 m) de N,N-diméthylformamide, refroidi à -5°C, on ajoute lentement 1,27 ml (0,0139 m) d'oxychlorure de phosphore. Dès que l'addition est terminée, on lave avec 0,6 ml de tétrahydrofurane. On maintient sous agitation à -5°C pendant 15 minutes, puis on ajoute au mélange, en 30 minutes, une solution de 5 g (0,011 m) d'acide (Z)-2-(2-benzyloxycarbonylaminothiazol-4-yl)-2-(tert-butoxycarbonylméthoxyimino)acétique monohydraté (PREPARATION) dans 50 ml de tétrahydrofurane. On maintient le mélange à -5°C pendant 15 minutes, puis on y ajoute, en 10 minutes, 4,7 g (0,0103 m) de p-toluènesulfonate de 7-amino-3-vinyl-3-céphème-4-carboxylate de tert-butyle. On agite le mélange à -5°+0°C pendant 90 minutes et, après un contrôle éventuel par HPLC, on verse la masse réactionnelle dans un mélange de 4,5 g de bicarbonate de sodium, 280 ml d'eau et 145 ml de dichlorométhane. On agite pendant 15 minutes, puis on separe les deux phases. La phase aqueuse est éliminée, la phase organique est lavée avec 50 ml d'eau, séchée sur du sulfate de magnésium et évaporée jusqu'à l'obtention d'une huile. On reprend le résidu avec 125 ml d'éther ethylique et, après 30 minutes, on filtre, on lave avec de l'éther de petrole et on sèche sous vide à 30°C. On obtient ainsi 6,56 g (91%) de 7β-[(Z)-2-(2-benzyloxycarbonylaminothiazol-4-yl)-2-(tert-butoxycarbonylméthoxyimino)acétamido]-3-vinyl-3-céphèm-4-carboxylate de tert-butyle. P.f.: 74ö76°C. IR:νₘₐₓ (KBr): 3266, 2978, 2933, 1786, 1723, 1686, 1559 cm⁻¹.
¹H-RMN (CDCl₃): δ (p.p.m.) 1,41 (9H, s, -CMe₃), 1,53 (9H, s, -CMe₃), 3,56 (2H, ABq, J=17,8 Hz, CH₂-S), 4,69 (2H, m, OCH₂), 5,25 (2H, s, OCH₂-C₆H₅), 5,31 (1H, d, J=11,4 Hz, =CH₂ vinyle), 5,44 (1H, d, J=17,5 Hz, =CH₂ vinyle), 5,8 (1H, d, J=4,9 Hz, C₆-H), 5,94 (1H, dd, J=4,9 et 8,6 Hz, C₇-H), 7,08 (1H, dd, J=17,5 et 11,4 Hz, =CH vinyle), 7,33ö7,37 (6H, protons aromatiques et proton thiazole).

### EXEMPLE 2

(a) A un mélange de 95,6 ml de tétrahydrofurane et 9 ml (0,117 m) de N,N-diméthylformamide, refroidi à -5°C, on ajoute, en 30 minutes, une solution de 10 ml (0,109 m) d'oxychlorure de phosphore dans 5 ml de tétrahydrofurane, puis on agite pendant 15 minutes à -5°C et on ajoute enfin, en 30 minutes, une solution de 30 g (0,091 m) d'acide (Z)-2-(2-formamidothiazol-4-yl)-2-(tert-butoxycarbonylméthoxyimino) acétique dans 63 ml de tétrahydrofurane. Après 25 minutes d'agitation à -5°C, on ajoute au mélange, en 10 minutes et à -5ö0°C, 37,5 g (0,0825 m) de p-toluènesulfonate de 7-amino-3-vinyl-3-céphème-4-carboxylate de tert-butyle. On agite la masse réactionnelle 90 minutes à -5+0°C et, après un contrôle éventuel par HPLC pour vérifier que la réaction est complète, on la verse dans un mélange de 3 g de bicarbonate de sodium, 800 ml d'eau et 650 ml de dichlorométhane, la température restant à environ 20ö22°C et le pH à 1,7ö1,8. Après 15 minutes d'agitation, on laisse bien séparer les deux couches, puis on recueillit la phase organique, alors que la phase aqueuse est eliminée. On lave la phase organique avec 300 ml d'eau et on l'utilise pour l'isolement du produit y dissous ou pour la transformation de ce dernier dans le céfixime trihydraté.
(b) La phase organique obtenue dans l'étape (a) est concentrée sous vide jusqu'à l'obtention d'une huile qui est reprise dans l'éther isopropylique. On filtre et on sèche sous vide à 30°C. On obtient ainsi 47,0 g (96%) de 7β-[(Z)-2-(2-formamidothiazol-4-yl)-2-(tert-butoxycarbonylméthoxyimino)acétamido]-3-vinyl-3-céphème-4-carboxylate de tert-butyle. P.f.: 132+133°C.
   IR:νₘₐₓ (KBr): 3268, 2977, 2933, 1786, 1719, 1695, 1544, 1368 cm⁻¹. ¹H-RMN (CDCl₃): δ (p.p.m.): 1,42 (9H, s, -CMe₃), 1,53 (9H, s, -CMe₃), 3,54 (2H, ABq, J=17,6 Hz, CH₂S), 4,66 (2H, s, O-CH₂), 5,08 (1H, d, J=4,9 Hz, C₆-H), 5,31 (1H, d, J=11 Hz, =CH₂ vinyle), 5,42 (1H, d, J=18,1 Hz, =CH₂ vinyle), 5,91 (1H, dd, J=8,5 et 4,9 Hz, C₇-H), 7,06 (1H, dd, J=18,1 et 11 Hz, =CH vinyle), 7,47 (1H, s, proton thiazole), 8,58 (1H, s, OHC-).

### EXEMPLE 3

On concentre la phase organique, telle qu'obtenue à la fin de l'étape (a) de l'Exemple 2, jusqu'à un volume d'environ 80 ml. A la solution résiduelle, on ajoute 200 ml de méthanol et après, en 10 minutes et à +15°C, 22,5 ml d'acide chlorhydrique à 35%. Après 90 minutes, on contrôle éventuellement la fin de la réaction par HPLC et on verse le mélange réactionnel dans 800 ml d'eau. On ajuste le pH à 6,5 par addition d'environ 47 ml d'hydroxyde de sodium à 15% on poursuit l'agitation une heure à 20°C, on filtre, on lave trois fois avec 200 ml d'eau et on sèche sous vide à 35°C. On obtient ainsi 43,2 g (92%) de 7β-[(Z)-2-(2-aminothiazol-4-yl)-2-(tert-butoxycarbonylméthoxyimino)acétamido]-3-vinyl-3-céphème-4-carboxylate de tert-butyle. P.f.: 120ö121°C.
IR: νₘₐₓ: 3282, 2977, 2933, 1786, 1720, 1685, 1610, 1539 cm⁻¹. ¹H-RMN (CDCl₃): δ (p.p.m.) 1,41 (9H, s, -CMe₃), 1,52 (9H, s, -CMe₃), 3,54 (2H, ABq J=17,1 Hz, CH₂S), 4,68 (2H, ABq, J=16,9 Hz, O-CH₂), 5,06 (1H, d, J=4,9 Hz, C₆-H), 5,29 (1H, d, J=11,1 Hz, =CH₂ vinyle), 5,42 (1H, d, J=17,5 Hz, =CH₂ vinyle), 5,93 (1H, dd, J=8,6 et 4,9 Hz, C₇-H), 6,93 (1H, s, proton thiazole), 7,06 (1H, dd, J=17,5 et 11,1 Hz, =CH vinyle).

### EXEMPLE 4

A une suspension de 9,55 g (0,029 m) d'acide (Z)-2-(2-formamidothiazol-4-yl)-2-(tert-butoxycarbonylméthoxyimino)acétique in 120 ml de dichlorométhane on ajoute, à environ 0°C et en 5 minutes, 7,5 ml (0,054 m) de triéthylamine, en obtenant ainsi une solution limpide. Après refroidissement à -10°C, on ajoute goutte à goutte, une solution de 3,4 ml (0,044 m) de chlorure de méthanesulfonyle dans 5 ml de dichlorométhane. Après 15 minutes d'agitation à -10°C, on ajoute 12,72 g (0,028 m) de p-toluènesulfonate de 7-amino-3-vinyl-3-céphème-4-carboxylate de tert-butyle. On agite 4 heures à une température de -5°C à -10°C et on contrôle la fin de la réaction par HPLC, puis on verse le mélange réactionnel dans une solution aqueuse de bicarbonate de sodium à 5%. On agite pendant 15 minutes à 20°C, on laisse séparer les deux phases, on récupère la phase organique, on la lave avec 25 ml d'eau et on la concentre sous vide jusqu'à ce qu'on obtient une huile. On reprend cette huile avec 50 ml d'éther isopropylique, on filtre et on sèche sous vide à 30°C. On obtient ainsi 13,3 g (80%) de 7β-[(Z)-2-(2-formamidothiazol-4-yl)-2-(tert-butoxycarbonylméthoxyimino)acétamido]-3-vinyl-3-céphème-4-carboxylate de tert-butyle identique au produit de l'Exemple 2(b).

### EXEMPLE 5

A une suspension de 13,83 g (0,042 m) d'acide (Z)-2-(2-formamidothiazol-4-yl)-2-(tert-butoxycarbonylméthoxyimino)acétique in 132 ml de N,N-diméthylacétamide on ajoute, à 2°C, 8,0 g (0,042 m) de chlorure de p-toluènesulfonyle, puis 11,7 ml (0,084 m) de triéthylamine. Après 2 heures d'agitation à 0°C, on ajoute, à la solution limpide ainsi obtenue, 19,08 g (0,042 m) de p-toluènesulfonate de 7-amino-3-vinyl-3-céphème-4-carboxylate de tert-butyle et 0,3 g de 4-diméthylaminopyridine. On agite le mélange réactionnel pendant 5 heures tout en contrôlant le déroulement de la réaction par HPLC. Lors que la réaction est terminée, on verse le mélange dans 160 ml d'une solution aqueuse de bicarbonate de sodium à 5%, on extrait avec 150 ml de dichlorométhane et on lave avec de l'eau. On évapore le solvant jusqu'à l'obtention d'une huile et l'on opère comme décrit dans l'Exemple 4 pour isoler 21,6 g (86%) de 7β-f(Z)-(2-formamidothiazol-4-yl)-2-(tert-butoxycarbonylméthoxyimino)acétamido]-3-vinyl-3-céphème-4-carboxylate de tert-butyle identique au produit de l'Exemple 2(b).

### EXEMPLE 6

A 75 ml d'anisole on ajoute, en 30 minutes et à 25+30°C, 23 g (0,172 m) de trichlorure d'aluminium anhydre et on maintient le mélange sous agitation 15 minutes à 35°C. A la solution ainsi obtenue on ajoute 120 ml de dichlorométhane, puis on refroidit ledit mélange à 10°C et on y ajoute, en 60 minutes, une solution de 33 g (0,058 m) de 7β-[(Z)-(2-(2-aminothiazol-4-yl)-2-(tert-butoxycarbonylméthoxyimino)acétamido]-3-vinyl-3-céphème-4-carboxylate de tert-butyle, tel qu'obtenu dans l'Exemple 3, dans 140 ml de dichlorométhane. On agite le mélange à 10°C pendant 30 minutes, puis on verse la masse réactionnelle dans un mélange de 400 ml d'eau et 41,5 ml d'acide chlorhydrique à 35%. On amorce la cristallisation et on agite à 10°C pendant 60 minutes. On filtre le produit, on le lave avec du dichlorométhane et on le suspend, encore humide, dans 300 ml d'eau. On ajuste le pH du mélange à 6,5 par addition d'environ 36 ml d'hydroxyde de sodium à 15%, on ajoute de la Dicalite® 408, on filtre sur une couche de Diafioc® 40 et on lave avec 60 ml d'eau. A la solution ainsi filtrée, on ajoute 150 ml d'acétate d'éthyle, on ajuste le pH à 3,2 par addition d'environ 6 ml d'acide chlorhydrique à 15% et on amorce la cristallisation par addition d'un échantillon de céfixime trihydraté. On ajuste le pH à 2,5 par addition d'environ 6 ml d'acide chlorhydrique à 15%, on agite pendant une heure, puis on laisse reposer le mélange pendant 15 heures à 10ö15°C. Après cette période, le produit est filtré, lavé avec 50 ml d'eau et dissous dans l'eau par addition d'une solution d'hydroxyde de sodium jusqu'à pH 6,0ö6,2. La solution ainsi obtenue est introduit sur une colonne de résine Amberlite®XAD 1180. L'éluat est recueilli et additioné de l'acétate d'éthyle et le pH de la solution est ajusté à 3,0ö3,2 par addition d'acide chlorhydrique à 15%. On laisse amorcer la cristallisation pendant 30 minutes et ensuite, on ajuste le pH du mélange à 2,4ö2,5 avec HCl à 15%. Après une heure d'agitation à 5°C, on filtre le céfixime trihydraté ainsi obtenue qui est ensuite séchée sous vide à 35ö40°C. On obtient ainsi 25,9 g (88%) de céfixime trihydraté identique à un échantillon authentique.

### EXEMPLE 7

A 40 ml d'anisole on ajoute, à portions et à 25ö30°C, 17,5 g (0,131 m) de trichlorure d'aluminium anhydre, puis on agite le mélange pendant 105 minutes jusqu'à dissolution complète. On ajoute 60 ml de dichlorométhane, puis on refroidit le mélange à 10°C et on y ajoute une solution de 20,4 g (0,0291 m) de 7β-[(Z)-2-(2-benzyloxycarbonylaminothiazol-4-yl)-2-(tert-butoxycarbonylméthoxyimino)acétamido]-3-vinyl-3-céphèm-4-carboxylate de tert-butyle, tel qu'obtenu dans l'Exemple 1. On laisse le mélange à 10°C pendant environ 75 minutes et, après avoir éventuellement contrôlé la fin de la réaction par HPLC, on verse la masse réactionnel dans 200 ml d'eau contenant 20,8 ml d'acide chlorhydrique à 35%. On amorce la cristallisation et on agite une heure à 10°C. On filtre le chlorhydrate cristallin ainsi obtenu et on lave avec 30 ml de dichlorométhane. On suspend le produit humide dans 150 ml d'eau, on ajuste le pH à 6,5 par addition d'environ 18 ml d'hydroxyde de sodium à 15%, on ajoute de la Dicalite ®408, on filtre sur une couche de Diafioc®40 et on lave avec 30 ml d'eau. A la solution limpide ainsi obtenue on ajoute 80 ml d'acétate d'éthyle, on ajuste le pH à 3,2 avec de l'acide chlorhydrique à 15% et on amorce la cristallisation par addition d'un échantillon de céfixime trihydraté. On agite pendant 30 minutes, puis on ajuste le pH à 2,5 avec de l'acide chlorhydrique à 15%. On maintient le mélange 15 heures à 5°C, puis on filtre, on lave avec de l'eau froide, on dissout le produit dans l'eau par addition d'hydroxyde de sodium à 15% jusqu'à pH 6,0ö6,2. On charge la solution ainsi obtenue sur une colonne de résine Amberlite ®XAD 1180, on élue et on recueillite le fractions riches, on ajoute de l'acétate d'éthyle et on ajuste le pH de la solution ainsi obtenue à 3,0ö3,2 par addition d'acide chlorhydrique à 15%. On laisse amorcer la cristallisation pendant 30 minutes et, ensuite, on ajuste le pH du mélange à 2,4ö2,5 avec de l'acide chlorhydrique à 15%. Après une heure d'agitation à 5°C, on filtre le produit qui est ainsi cristallisé et on le sèche sous vide à 35°C. On obtient ainsi 13,27 g (90,2%) de cèfixime trihydraté identique à un échantillon authentique.

## Revendications

1. Procédé pour la préparation du céfixime trihydraté de formule (A) 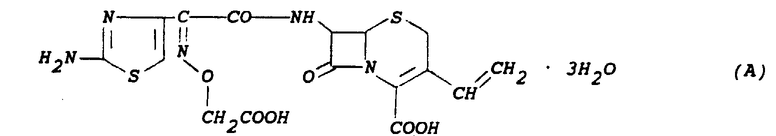 caractérisé en ce que
(a) on traite un dérivé fonctionnel de l'acide (Z)-2-(2-aminothiazol-4-yl)-2-(tert-butoxycarbonylméthoxyimino)acétique N-protégé de formule (I) 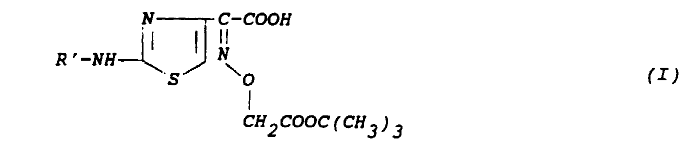 dans laquelle R' représente un groupe N-protecteur, avec le 7-amino-3-vinyl-3-céphème-4-carboxylate de tert-butyle de formule (II) 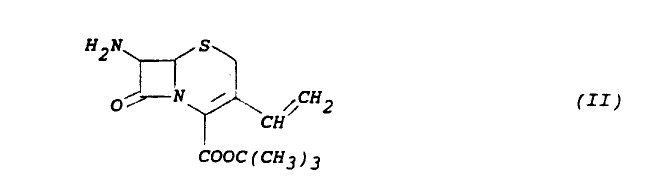 ou avec un de ses sels; et
(b) après l'élimination éventuelle du groupe N-protecteur du produit ainsi obtenu de formule (III) 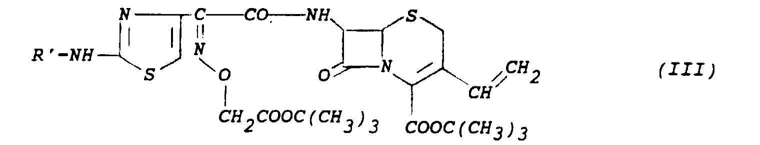 dans laquelle R' est tel que défini ci-dessus, on traite le composé ainsi obtenu de formule (IV) 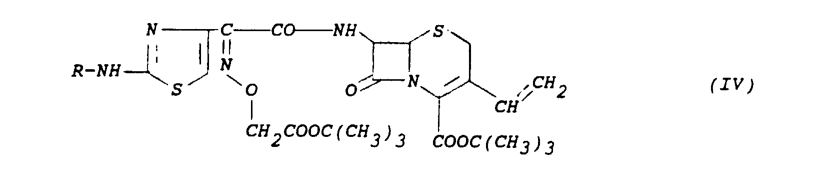 dans laquelle R représente l'hydrogène ou R', avec trichlorure d'aluminium et anisole.

2. Procédé selon la revendication 1, caractérisé en ce que comme produit de départ on utilise un composé de formule (I) dans laquelle R' est un groupe formyle, trichloroacétile, trityle, benzyloxycarbonyle ou tert-butoxycarbonyle.

3. Procédé selon la revendication 2, caractérisé en ce que comme produit de départ on utilise un composé de formule (I) dans laquelle R' est formyle.

4. Procédé selon la revendication 3, caractérisé en ce que dans l'étape (b):
(b₁) le composé de formule (III), dans laquelle R' est formyle, est soumis à une N-déprotection par hydrolyse avec de l'acide chlorhydrique dans le méthanol pour obtenir un composé de formule (IV) où R est l'hydrogène; et
(b₂) ce dernier est soumis à l'action du trichlorure d'aluminium et de l'anisole.

5. Procédé selon la revendication 2, caractérisé en ce que comme produit de départ on utilise un composé de formule (I) dans laquelle R' est benzyloxycarbonyle.

6. Procédé selon la revendication 5, caractérisé en ce que, dans l'étape (b), le composé de formule (III), dans laquelle R' est benzyloxycarbonyle, est directement soumis à l'action du trichlorure d'aluminium et de l'anisole.

7. Procédé selon la revendication 1, caractérisé en ce que le réactif de formule (II) est utilisé sous forme de p-toluènesulfonate.

8. Procédé selon une des revendications 1 à 7, caractérisé en ce que comme dérivé fonctionnel de l'acide de formule (I) on utilise un anhydride mixte avec un acide sulfonique.

9. Procédé selon la revendication 8, caractérisé en ce que ledit acid sulfonique est l'acide méthanesulfonique ou p-toluènesulfonique.

10. Procédé selon une des revendications 1 à 7, caractérisé en ce que comme dérivé fonctionnel de l'acide de formule (I) on utilise le chlorure.

11. Procédé selon la revendication 10, caractérisé en ce que ledit chlorure est préparé "in situ" par réaction de l'acide de formule (I) avec oxychlorure de phosphore et N,N-diméthylformamide.

12. Procédé selon la revendication 1, caractérisé en ce que, dans l'étape (b), le composé de formule (IV) est préalablement soumis à une N-déprotection.

13. Composé de formule (IV) 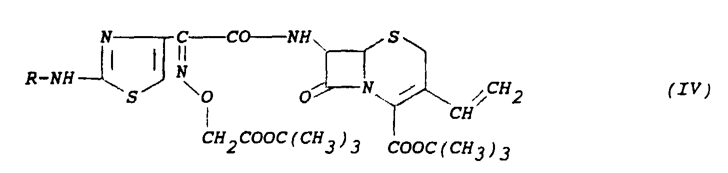 dans laquelle R est l'hydrogène ou un groupe N-protecteur, choisi parmi les groupes formyle, trichloroacétile, trityle, benzyloxycarbonyle et tert-butoxycarbonyle.

14. Le 7β-[(Z)-2-(2-aminothiazol-4-yl)-2-(tert-butoxycarbonylméthoxyimino)acétamido]-3-vinyl-3-céphème-4-carboxylate de tert-butyle.

15. Le 7β-[(Z)-2-(2-formamidothiazol-4-yl)-2-(tert-butoxycarbonyl*méthoxyimino*)acétamido]-3-vinyl-3-céphème-4-carboxylate de tert-buty le.

16. Le 7β-((Z)-2-(2-benzyloxycarbonylaminothiazol-4-yl)-2-(tert-butoxycarbonylméthoxyimino)acétamido]-3-vinyl-3-céphème-4-carboxylate de tert-butyle.

## Patentansprüche

1. Verfahren zur Herstellung von Cefiximtrihydrat der Formel (A): 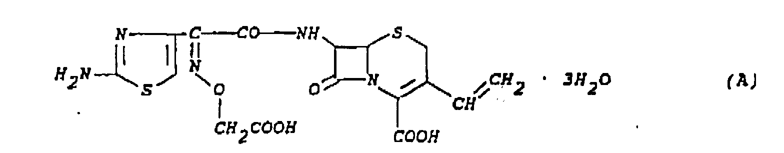 dadurch gekennzeichnet, daß
(a) ein funktionelles Derivat der N-geschützten (Z)-2-(2-Aminothiazol-4-yl)-2-(tert-butoxycarbonylmethoxyimino)essigsäure der Formel (I) 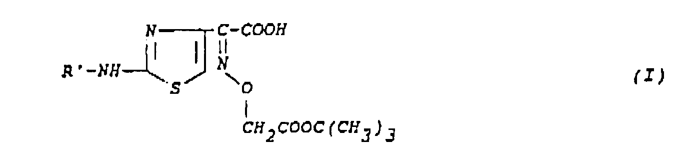 worin R' eine N-Schutzgruppe darstellt, mit 7-Amino-3-vinyl-3-cephem-4-carbonsäure-tert-butylester der Formel (II) 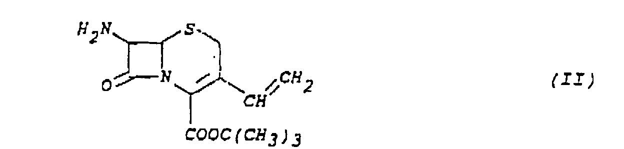 oder mit einem seiner Salze behandelt wird; und
(b) nach der eventuellen Abspaltung der N-Schutzgruppe des so erhaltenen Produktes der Formel (III) 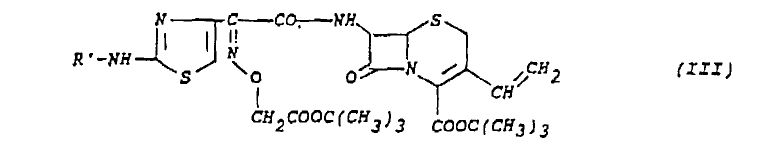 worin R' wie oben definiert ist, die so erhaltene Verbindung der Formel (IV) 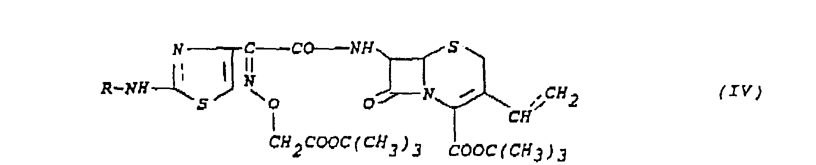 worin R Wasserstoff oder R' darstellt, mit Aluminiumtrichlorid und Anisol behandelt wird.

2. Verfahren gemäß Anspruch 1, dadurch gekennzeichnet, daß als Ausgangsmaterial eine Verbindung der Formel (I) verwendet wird, in der R' eine Formylgruppe, eine Trichloracetylgruppe, eine Tritylgruppe, eine Benzyloxycarbonylgruppe oder eine tert-Butoxycarbonylgruppe ist.

3. Verfahren gemäß Anspruch 2, dadurch gekennzeichnet, daß als Ausgangsmaterial eine Verbindung der Formel (I) verwendet wird, in der R' eine Formylgruppe ist.

4. Verfahren gemäß Anspruch 3, dadurch gekennzeichnet, daß im Schritt (b)
(b1) die Verbindung der Formel (III), in der R' eine Formylgruppe ist, einer N-Entschützung durch Hydrolyse mit Chlorwasserstoffsäure in Methanol unter Erhalt einer Verbindung der Formel (IV), in der R Wasserstoff ist, unterworfen wird; und
(b2) letztere der Wirkung von Aluminiumtrichlorid und Anisol unterworfen wird.

5. Verfahren gemäß Anspruch 2, dadurch gekennzeichnet, daß als Ausgangsmaterial eine Verbindung der Formel (I) verwendet wird, in der R' eine Benzyloxycarbonylgruppe ist.

6. Verfahren gemäß Anspruch 5, dadurch gekennzeichnet, daß im Schritt (b) die Verbindung der Formel (III), in der R' eine Benzyloxycarbonylgruppe ist, direkt der Wirkung von Aluminiumtrichlorid und Anisol unterworfen wird.

7. Verfahren gemäß Anspruch 1, dadurch gekennzeichnet, daß das Reagens der Formel (II) in Form des p-Toluolsulfonats verwendet wird.

8. Verfahren gemäß einem der Ansprüche 1 bis 7, dadurch gekennzeichnet, daß als funktionelles Derivat der Säure der Formel (I) ein gemischtes Anhydrid mit Sulfonsäure verwendet wird.

9. Verfahren gemäß Anspruch 8, dadurch gekennzeichnet, daß die Sulfonsäure Methansulfonsäure oder p-Toluolsulfonsäure ist.

10. Verfahren gemäß einem der Ansprüche 1 bis 7, dadurch gekennzeichnet, daß als funktionelles Derivat der Säure der Formel (I) das Chlorid verwendet wird.

11. Verfahren gemäß Anspruch 10, dadurch gekennzeichnet, daß das Chlorid "in situ" durch Reaktion der Säure der Formel (I) mit Phosphoroxychlorid und N,N-Dimethylformamid hergestellt wird.

12. Verfahren gemäß Anspruch 1, dadurch gekennzeichnet, daß im Schritt (b) die Verbindung der Formel (IV) vorher einer N-Entschützung unterworfen wird.

13. Verbindung der Formel (IV) 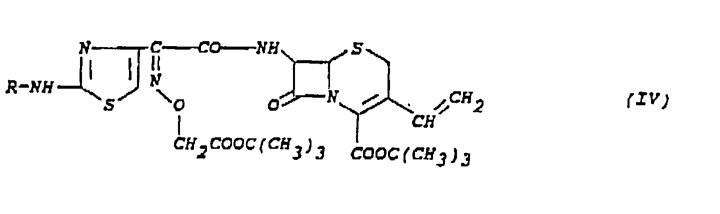 worin R Wasserstoff oder eine N-Schutzgruppe, ausgewählt aus Formylgruppe, Trichloracetylgruppe, Tritylgruppe, Benzyloxycarbonylgruppe und tert-Butoxycarbonylgruppe, ist.

14. 7β-[(Z)-2-(2-aminothiazol-4-yl)-2-(tert-butoxycarbonylmethoxyimino)acetamido]-3-vinyl-3-cephem-4-carbonsäure-tert-butylester.

15. 7β-[(Z)-2-(2-formamidothiazol-4-yl)-2-(tert-butoxycarbonylmethoxyimino)acetamido]-3-vinyl-3-cephem-4-carbonsäure-tert-butylester.

16. 7β-[(z)-2-(2-benzyloxycarbonylaminothiazol-4-yl)-2-(tert-butoxycarbonylmethoxyimino)acetamido]-3-vinyl-3-cephem-4-carbonsäure-tert-butylester.

## Claims

1. Process for the preparation of cefixime trihydrate of formula (A) 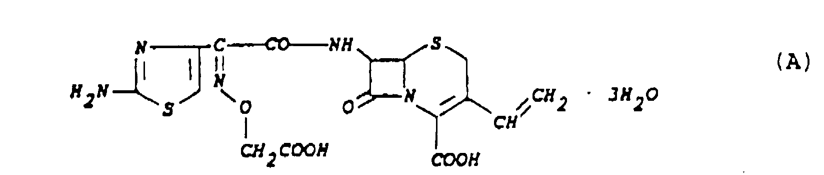 characterised in that
(a) a functional derivative of N-protected (Z)-2-(2-aminothiazol-4-yl)-2-(tert-butoxycarbonylmethoxyimino)acetic acid of formula (I) 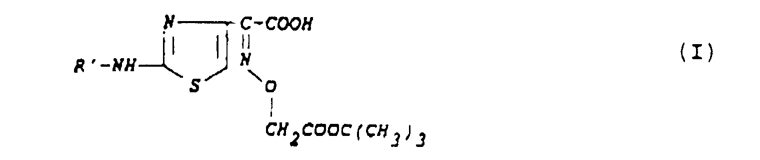 wherein R' represents an N-protective group, is treated with tert-butyl-7-amino-3-vinyl-3-cephem-4-carboxylate of formula (II) 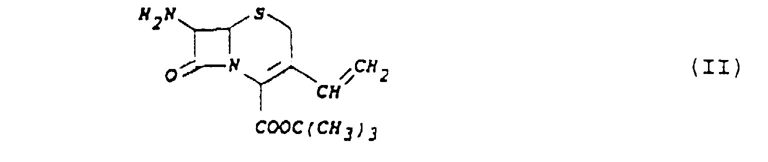 or with one of its salts; and
(b) after optional removal of the N-protective group of the product so obtained of formula (III) 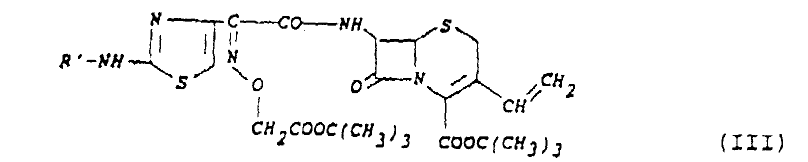 wherein R' is as defined above, the compound so obtained of formula (IV) 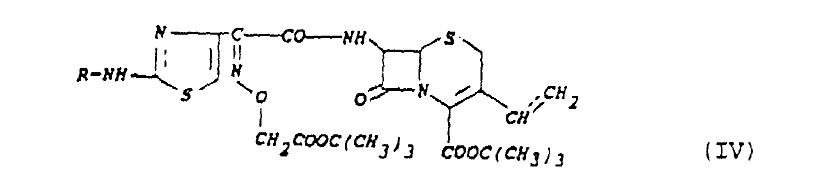 wherein R represents hydrogen or R', is treated with aluminium trichloride and anisole.

2. Process according to claim I, characterised in that a compound of formula (I) wherein R' is a formyl, trichloroacetyl, trityl, benzyloxycarbonyl or tert-butoxycarbonyl group is used as the starting material.

3. Process according to claim 2, characterised in that a compound of formula (I) wherein R' is formyl is used as the starting material.

4. Process according to claim 3, characterised in that in stage (b) ;
(b₁) the compound of formula (III), wherein R' is formyl, is subjected to N-deprotection by hydrolysis with hydrochloric acid in methanol to obtain a compound of formula (IV) wherein R is hydrogen; and
(b₂) the latter compound is subjected to the action of aluminium trichloride and anisole.

5. Process according to claim 2, characterised in that a compound of formula (I) wherein R' is benzyloxycarbonyl is used as the starting material.

6. Process according to claim 5, characterised in that, in stage (b), the compound of formula (III), wherein R' is benzyloxycarbonyl, is subjected directly to the action of aluminium trichloride and anisole.

7. Process according to claim 1, characterised in that the reagent of formula (II) is used in the form of p-toluenesulphonate.

8. Process according to any one of claims 1 to 7, characterised in that a mixed anhydride with a sulphonic acid is used as the functional derivative of the acid of formula (I).

9. Process according to claim 8, characterised in that the sulphonic acid is methanesulphonic or p-toluenesulphonic acid.

10. Process according to any one of claims 1 to 7, characterised in that chloride is used as the functional derivative of the acid of formula (I).

11. Process according to claim 10, characterised in that the chloride is prepared in situ by reaction of the acid of formula (I) with phosphorus oxychloride and N,N-dimethylformamide.

12. Process according to claim 1, characterised in that, in stage (b), the compound of formula (IV) is previously subjected to N-deprotection.

13. Compound of formula (IV) 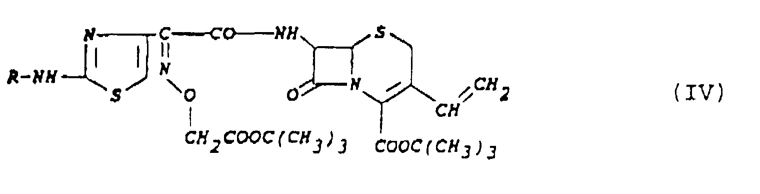 wherein R is hydrogen or an N-protective group selected from the groups formyl, trichloroacetyl, trityl, benzyloxycarbonyl and tert-butoxycarbonyl.

14. Tert-butyl-7β-[(Z)-2-(2-aminothiazol-4-yl)-2-(tert-butoxycarbonylmethoxyimino)acetamido]-3-vinyl-3-cephem-4-carboxylate.

15. Tert-butyl-7β-[(Z)-2-(2-formamidothiazol-4-yl)-2-(tert-butoxycarbonylmethoxyimino)acetamido]-3-vinyl-3-cephem-4-carboxylate.

16. Tert-butyl-7β-[(Z)-2-(2-benzyloxycarbonylaminothiazol-4-yl)-2-(tert-butoxycarbonylmethoxyimino)acetamido]-3-vinyl-3-cephem-4-carboxylate.
